(19) Europäisches Patentamt
European Patent Office
Office européen des brevets

(11) **EP 2 548 458 B1**

(12) **EUROPEAN PATENT SPECIFICATION**

(45) Date of publication and mention of the grant of the patent:
**03.07.2013 Bulletin 2013/27**

(51) Int Cl.:
*A23L 1/305* [(2006.01)]   *C12P 21/06* [(2006.01)]

(21) Application number: **11175068.3**

(22) Date of filing: **22.07.2011**

(54) **Lupin-derived compounds having hypotensive activity and process for their production**

Lupinen-abgeleitete Verbindungen mit hypotonischer Wirkung und Verfahrensprozess zu ihrer Herstellung

Composés dérivés de lupin dotés d'une activité hypotensive et leur procédé de production

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**23.01.2013 Bulletin 2013/04**

(73) Proprietor: HPF Nutraceutics S.r.L.
**20142 Milan (IT)**

(72) Inventors:
• **Arnoldi, Anna**
  **20131 Milano (IT)**
• **Boschin, Giovanna**
  **20047 Brughero (Milan) (IT)**
• **Resta, Donatella**
  **20060 Gessate (Milano) (IT)**
• **Scigliuolo, Graziana**
  **20161 Milano (IT)**

(74) Representative: **Lunati & Mazzoni S.r.L.**
**Via Carlo Pisacane, 36**
**20129 Milano (IT)**

(56) References cited:
**EP-A1- 1 661 913        WO-A2-2007/004876**
**KR-B1- 100 818 010      US-A1- 2005 142 166**
**US-A1- 2008 050 458**

• **ELENA SIRTORI A, DONATELLA RESTA, FRANCESCA BRAMBILLA, CHRISTIAN ZACHERL, ANNA ARNOLDI: "The effects of various processing conditions on a protein isolate from Lupinus angustifolius", FOOD CHEMISTRY, vol. 120, 2010, pages 496-504, XP002664537, DOI: 10.1016/j.foodchem. 2009.10.043 Retrieved from the Internet: URL: http://hydh.hbstl.org.cn/uploadfiles/K SZJ5H7Y1304392429670.pdf> [retrieved on 2011-11-28]**

## Description

The present invention relates to lupin-derived compounds having hypotensive activity, and to a process for their production, as pointed out in the preamble of the first claim.

In particular, this process aims to obtain compounds with hypotensive activity, which are substantially made up of peptide mixtures derived from lupin proteins, having ACE-inhibitory activity, and suitable to be used, for example, for producing functional foods and drinks, and dietary supplements.

KR 100 818 010 B1 describes a lupin peptide extract comprising peptides of a molecular weight lower than 10,000 Da and US 2008/050458 A1 relates to a composition comprising a lupin total extract consisting of a lupin sugar extract and a lupin peptide extract.

It is known that actually, in order to prevent and treat disorders such as arterial hypertension, chronic cardiac failure or other problems connected with diabetes, drugs having ACE-inhibitory activity are used.

The mechanism of action of ACE-inhibitors is based on the reduction of the production of angiotensin II from angiotensin I, and by slowing down of bradykinin degradation through the inhibition of the so-called "angiotensin converting enzyme" (ACE). ACE performs two functions: a) converts angiotensin I into angiotensin II, an octapeptide vasoconstrictor, that causes pressure increase; b) degrades the bradykinin, which on the contrary is a vasodilator, into inactive products.

The ACE activity is concentrated on the three last amino acids of the peptidic chain of angiotensin I exhibiting the phenylalanine-histidine-leucine sequence at the carboxy-terminal end.

Traditionally, ACE-inhibiting drugs are produced through synthetic processes whereas, in recent years, the interest for nutraceutical compounds of animal or vegetable origin provided with similar activity, has become higher and higher. In particular, some researches have been dedicated to proteins or peptides that can be used in the formulation of functional foods, drinks or dietary supplements. In particular, peptides having ACE-inhibitory activity are mainly obtained by processing casein, as described in patents JP2009073765, JP2007217406 and EP1374885 or, alternatively, by processing other animal or plant proteins.

Actually, for producing functional foods and drinks and dietary supplements having ACE-inhibitory activity, the research is addressing its attention to processing extracts from plants and, in particular, from legumes, such as *Glycine max,* i.e. soybean, that have been proved to possess very interesting properties.

The known art mentioned above has some important drawbacks.

In fact, due to the increasing production of genetically modified soybean, the use of this seed as starting material is limited because European consumers have a very negative and distrustful attitude towards organisms that could be genetically modified.

Another problem of soybean is represented by the reduced ACE-inhibitory activity of the peptides obtained therefrom, making processing thereof not always advantageous above mainly from the economical point of view.

The technical task underlying the present invention is to conceive lupin-derived compounds having hypotensive activity and a process for their production able of substantially solving the above mentioned drawbacks.

Within the scope of this technical task, an important aim of the invention is to obtain a simple and easy manufacturing process.

Another important aim of the invention is to obtain compounds having hypotensive activity, which can be easily processed, are inexpensive and characterised by high ACE-inhibitory activity.

The technical task mentioned and the aims specified are achieved by lupin-derived compounds having hypotensive activity and a process for their production as claimed in the annexed claim 1.

Preferred embodiments are highlighted in the subclaims.

The features and advantages of the invention are hereinafter clarified by the detailed description of some preferred embodiments of the invention, with reference to the accompanying drawings, in which:

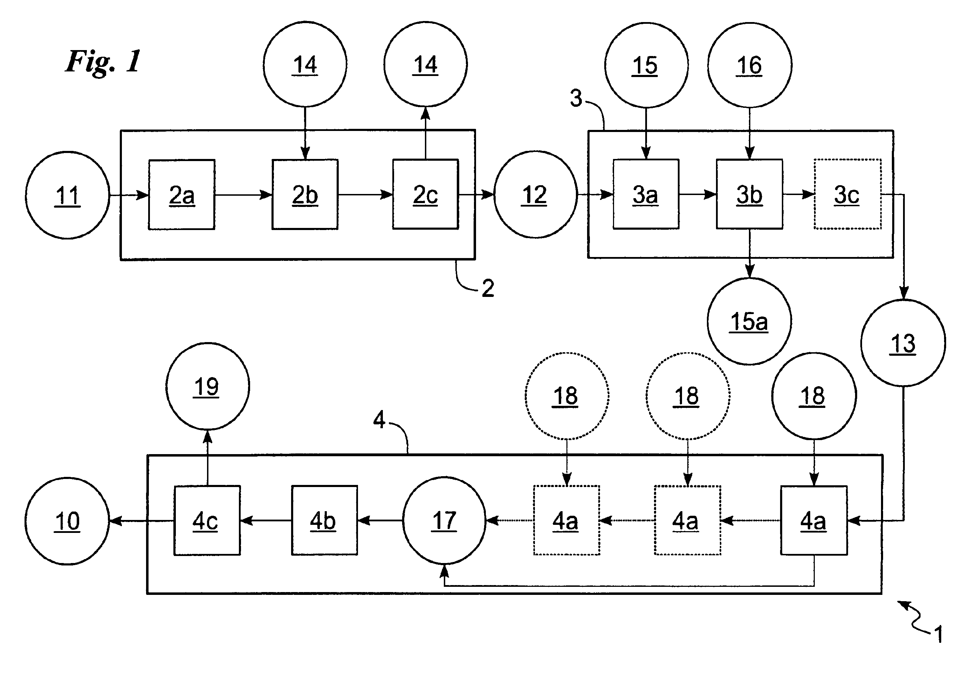

Fig. 1 is a diagram of the production process of compounds having hypotensive activity according to the invention;
Fig. 2a reproduces a graph giving an evaluation of the ACE inhibitory activity of an exemplary compound obtained by the production process of the invention; and
Fig. 2b shows another graph giving an evaluation of the ACE inhibitory activity of another exemplary compound.

With reference to the drawings, the production process of compounds having hypotensive activity according to the invention is generally identified with reference numeral 1.

It enables the production, by processing lupin seeds, of compounds having hypotensive activity 10, which mainly comprise active peptide mixtures characterised by high ACE-inhibitory activity.

In other words, process 1 includes processing of Lupinus seeds 11, commonly referred to as lupin, a plant belonging to the *Leguminosae* or Fabaceae order, *Papilonaceae* family, *Genestae* section, *Lupinus* genus. Preferably, the production process 1 includes processing of seeds 11 of the species *Lupinus albus* (white lupin), *Lupinus angustifolius* (narrow-leaf lupin), *Lupinus luteus* (yellow lupin) and *Lupinus mutabilis* (Andean lupin) and more preferably of the *Lupinus albus* or *Lupinus angustifolius* species.

Briefly, the production process **1** comprises a preparation step **2,** in which a defatted flour **12** is obtained from the Lupinus seeds 11; an extraction step **3** in which a highly-concentrated protein solution **13** is obtained from the defatted flour 12; and a hydrolysis step **4** in which the proteins contained in the protein solution 13 are partly fragmented in order to obtain the compounds having hypotensive activity 10.

The preparation step 2 includes an expansion sub-step **2a** in which, once possible impurities have been eliminated, seeds 11 are processed or worked to make the following processing operations more efficient.

In detail, in this expansion sub-step 2a the contact surface of the Lupinus seeds 1, i.e. the contact surface substantially identified by the external surface of the seeds, is increased.

This sub-step 2a can therefore be obtained through a flaking operation i.e. a process in which the Lupinus seeds 11 are heated and then compressed so as to reduce them into flakes.

Alternatively, the expansion sub-step 2a can be carried out through a grinding operation in which the seeds 11 are reduced into flour and then sieved in order to eliminate possible extraneous bodies and obtain a flour having a homogeneous granular size.

When sub-step 2a has been completed, the preparation step 2 includes a defatting sub-step **2b** in which the defatted flour 12 is obtained by extracting almost completely the lipids present in the Lupinus seeds 11.

This defatting sub-step 2b can be accomplished through shaking/stirring of a suspension made up of the Lupinus seeds 11 (as flour or flakes) and a solvent **14** or, alternatively, through percolation of the solvent 14 through the Lupinus seeds 11.

In the defatting sub-step 2b a solvent 14 characterised by a low dielectric constant is preferably used, which solvent therefore allows the lipids to be easily brought to a solution state, as lipids are nonpolar or have low polarity and therefore are not hydrosoluble. In particular, this solvent 14 is an organic solvent and preferably solvent 14 is hexane.

In this defatting sub-step 2b, the ratio of the weight "w" expressed in kg of the treated Lupinus seeds 14 to the volume v expressed in L (litres) of solvent 14 used is substantially included between 1/10 w/v and 1/50 w/v and more preferably it is substantially equal to 1/20 w/v or 1/20 kg/L.

In detail, the defatting sub-step 2b is substantially obtained at room temperature and over a period of time of substantially 24 hours.

When the defatting sub-step 2b has been completed, the preparation step 2 includes an elimination sub-step **2c** in which solvent 14 is separated from the flour or flakes derived from the Lupinus seeds 11 and therefore the defatted flakes or flour 12 are obtained.

The elimination sub-step 2c can be obtained through centrifugation, i.e. the suspension is centrifuged, or alternatively through filtering.

When the preparation step 2 has been completed, the production process 1 includes the extraction step 3 in which the protein solution 13 is obtained from defatted flour 12. The expression "protein solution" identifies a solution mainly of globular proteins.

In greater detail, in the extraction step 3, a protein solution 13 is obtained; it contains almost mainly the globular proteins, also referred to as conglutins, present in the Lupinus seed 11 and more specifically the protein solution 13 contains alpha, beta, gamma and delta conglutin.

This extraction step 3 therefore includes a protein-isolation sub-step **3a** in which the defatted flour 12 is treated with a weakly basic saline solution promoting solubilisation of the globular proteins increasing the ionic strength of the extraction environment.

In the isolation sub-step 3a, the defatted flour 12 is mixed with a basic saline water solution 15 which is preferably weakly basic, in a mixing ratio of flour 12 to solution 15 substantially included between 1/10 w/v and 1/50 w/v and preferably substantially equal to 1/30 w/v.

The basic saline solution 15 can, for example, consist of a weakly basic solution of TRIS-HCl (tris(hydroxymethyl) aminomethane chlorohydrate) with addition of a salt, NaCl for example, and therefore this solution 15 can be a solution of 100 mM of TRIS-HCl pH 8.0, 0.5 M NaCl.

In particular, the protein-isolation sub-step 3a is obtained at a temperature substantially included between 0°C and 30°C over a period of time almost included between 10 hours and 1 hour. Preferably it is obtained at a temperature substantially included between 15°C and 0°C for a time of substantially 1-5 hours. More preferably, the protein-isolation sub-step 3a is obtained at a temperature of substantially 4°C and for a time of substantially 2 hours.

Once the protein-isolation sub-step 3a has been completed, the extraction step 3 can include a refining sub-step **3b** adapted to complete formation of the protein solution 13 through elimination of possible undesirable substances 15a such as the salt residues of the saline solution 15 used in the previous sub-step 3a.

This refining sub-step 3b can therefore be obtained by dialysis, i.e. by treating the protein solution 13 with a dialysis solution **16** substantially similar to that used in sub-step 3a, but without the saline content. In particular, the dialysis solution 16 can be a 100 nM TRIS-HCl pH 8.0 solution.

After the refining sub-step 3b, the protein solution 13 is therefore completed and is ready for use in step 4 in which, through enzymatic hydrolysis of the protein solution 13, the compounds having hypotensive activity 10 are obtained, i.e. an active peptide mixture characterised by the almost exclusive presence of molecules having a molecular weight not exceeding 3000 Da. In particular, the peptide mixture, and therefore the compounds 10, have, as better described in the following, an $IC_{50}$ (i.e. a concentration value able to give inhibition of the ACE enzyme of 50%) lower than 1000 μg/mL, preferably lower than 300 μg/mL and more preferably lower than 200 μg/mL.

Once the refining sub-step 3b has been completed, the extraction step 3 can include a purification sub-step 3c in order to obtain a protein solution 13 consisting of further purified protein fractions, i.e. enriched fractions as regards the individual components constituting the mixture (alpha, beta, gamma, delta conglutin). In particular, this purification sub-step 3c can be obtained through physico-chemical processes (such as selective precipitation, centrifugation, separation), and chromatography.

The hydrolysis step 4 includes one or more enzymatic-hydrolysis sub-steps **4a** at the end of which a peptide solution **17** is obtained in which the proteins contained in the protein solution 13 have been hydrolysed into peptides, i.e. shorter amino acid chains and, more specifically, chains consisting of a lower number of amino acids. In detail, the hydrolysis step 4 can include a single enzymatic hydrolysis sub-step 4a or, alternatively, a plurality of enzymatic hydrolysis sub-steps 4a carried out in series.

The expression "enzymatic hydrolysis" identifies a chemical scission in which some enzymes **18** through a catalytic action cause the cleavage of the peptidic bonds, molecular bonds belonging to the proteins of the protein solution 13 and therefore promote degradation of the proteins into shorter amino acid chains, i.e. peptides.

This ability of breaking the bonds is caused in each enzyme 18 by the presence of the catalytic site or active site defining the enzyme portion directly concerned with the catalysis process of breaking the peptidic bonds that link the amino acids inside the protein sequence.

Therefore, in order to obtain the correct and desired protein fragmentation, in each enzymatic-hydrolysis sub-step 4a the protein solution 13 is submitted to the action of a specific enzyme different from one sub-step to the other and adapted to enable the globular proteins present in the protein solution to be broken up in order to obtain peptides of the desired molecular weight and ACE-inhibitory activity.

In detail, in the enzymatic-hydrolysis sub-step 4a the enzymes belonging to the hydrolase classes and in particular proteolytic enzymes are used as enzymes 18. In detail, the proteolytic enzymes are enzymes that are able to catalyse the cleavage of the peptidic bond between the amino group and the carboxy group of two consecutive amino acids within a protein sequence. They act by breaking up internally the polypeptidic chain of the protein, and, in this case, they are referred to as endopeptidases, or by starting from one end, and in this case they are referred to as aminopeptidases or carboxypeptidases.

Preferably, the enzymes 18 are endopeptidases, i.e. enzymes acting by breaking up internally the polypeptidic chain so as to break the peptidic bonds inside the protein molecule thus producing peptides of various length and therefore of appropriate molecular weight; however, they can also be aminopeptidases and carboxypeptidases. These enzymes can be extracted from natural sources, specifically from animals, plants or microganisms, or be synthesised (bio-technological or recombinant enzymes); in addition they can be soluble or immobilised on a solid support.

Therefore among the endopeptidases, enzymes such as pepsin, chymotrypsin, trypsin or enzyme mixtures such as Corolase® PP can be used. Pepsin and chymotripsin are adapted to mainly break the peptidic bonds involving aromatic amino acids causing their separation; trypsin is adapted to cleave mainly the peptidic bonds involving basic amino acids causing cleavage of the protein sequence.

Preferably, the enzymes 18 used are pepsin, trypsin and chymotrypsin and in particular pepsin, as highlighted by the evaluation process set out below.

This endopeptidase selection and in particular pepsin, trypsin and chymotrypsin, causes breaking of the peptidic bond to take place after the hydrophobic and aromatic amino acids generating peptides whose features are particularly suitable for interacting with the catalytic site of ACE and therefore for inhibiting the same, as proved by proper experiments and evaluations performed by the Applicant and reproduced here below.

In particular, these enzymes 18, by fragmentation of the protein chain at the hydrophobic and aromatic amino acids, enable to obtain peptides having a hydrophobic and/or aromatic amino acid as the terminal group, i.e. having an hydrophobic and/or aromatic amino acid as the last amino acid of the peptide chain. Preferably, the terminal group can consist of proline, tryptophan, phenylalanine or tyrosine.

In order to promote the activity of enzymes 18, in each enzymatic-hydrolysis sub-step 4a the protein solution 13 is brought to specific physico-chemical conditions as a function of the used enzyme.

In detail, solution 13 has, , a pH substantially equal to 2, using pepsin, while in, the pH is substantially equal to 8 using trypsin, chymotrypsin and Corolase® PP. The temperature of the protein solution 13 is substantially included between 20°C and 70°C, and in particular substantially included between 30°C and 40°C and, more specifically, the protein solution 13 has a temperature of substantially 37°C.

Finally, in order to ensure correct fragmentation in each enzyme hydrolysis sub-step 4a, optimized ratios and time of each individual sub-step have been suitably identified through a great number of experiments performed by the Applicant.

In detail, an optimal ratio expressed in weight/weight (kg/kg) has been identified between the protein solution 13 and enzyme 18 which is substantially included between 1/200 w/w and 1/20 w/w and preferably substantially corresponding to 1/100 w/w. On the contrary, the optimal time of reaction has been evaluated substantially included between 25 hours and 5 hours and preferably included between 20 hours and 10 hours. More preferably, each enzymatic-hydrolysis sub-step 4a has a duration of substantially 16 hours.

In particular the enzymatic-hydrolysis process, performed in all the provided sub-steps 4a has been considered as completed when in the peptide solution 17, molecules with a molecular mass exceeding 10000 Da or, in particular not exceeding 5000 Da, are not present.

Once all the enzymatic-hydrolysis sub-steps 4a have been completed, the hydrolysis step 4 includes at least one inactivation sub-step 4b in which the previously used enzymes are inactivated, and a separation sub-step 4c in which the active peptide mixture having the above described molecular weight and ACE-inhibitory activity (i.e. the compounds having hypotensive activity 10) is separated from the peptide solution 17.

The inactivation sub-steps 4b are adapted to inactivate the used enzymes still in solution in an irreversible manner.

In detail, each inactivation sub-step 4b is adapted to inactivate at least one specific enzyme by, for example, varying the solution pH or temperature. More specifically, for inactivating pepsin, the solution has been brought to a basic pH, while for inactivating trypsin, chymotrypsin and Corolase® PP the solution temperature has been raised to 100°C.

Once all enzymes have been inactivated, the peptide solution 17 is submitted to the separation sub-step 4c in which the compounds having hypotensive activity 10 are obtained; these compounds almost exclusively consist of peptides having molecular weight not exceeding 3000 Da and, as better described in the following examples, an $IC_{50}$ lower than 1000 $\mu$g/mL, preferably lower than 300 $\mu$g/mL and more preferably lower than 200 $\mu$g/mL. In other words, at the end of this sub-step 4c there are compounds having hypotensive activity that are able to give a 50% inhibition of ACE if present in a concentration expressed in weight/volume lower than 1000 $\mu$g/mL, preferably lower than 300 $\mu$g/mL, and more preferably lower than 200 $\mu$g/mL.

In the separation sub-step 4c, the peptide solution 17 is submitted to an ultrafiltration process and in particular to an ultrafiltration process obtained through a semipermeable membrane characterised by pores having a molecular cut-off substantially of 3000 Da.

In detail, in the separation sub-step 4c, the peptide solution 17 is submitted to an ultrafiltration process on 3000 Da molecular cut-off filters in a centrifuge , in which the solution 17 is divided into a waste suspension 19 consisting of peptides of molecular weight higher than 3000 Da and possibly proteins and/or whole enzymes (Retentate) and a peptide solution of molecular weight not exceeding 3000 Da and with $IC_{50}$ lower than 300 $\mu$g/mL, which therefore constitutes the compounds having hypotensive activity 10.

Once the separation sub-step 4c has been completed, the hydrolysis step 4 is completed and the production process 1 can include a further step, not shown in the figure, adapted to treat the compounds having hypotensive activity 10, in order to make them suitable for subsequent use, as an ingredient for producing foods, drinks or dietary supplements.

In detail, in this step the compounds having hypotensive activity 10 can be submitted to pasteurisation, UHT treatment and/or drying, so as to reduce the compounds having hypotensive activity 10 to a powder, easily usable in the food field for preparation of biscuits, pastries or any other food, drink or dietary supplement.

In order to evaluate the ACE-inhibitory activity of the compounds having hypotensive activity 10, they have been submitted to a suitable evaluation process worked out by the Applicant to determine the aforesaid ACE-inhibitory activity.

The evaluation process is based on the simulation of the angiotensin I through use of substrate, i.e. HHL the pseudo-tripeptide N-hippuril-histidyl-leucine (N-hippuril-histidyl-leucine hydrate).

In detail, in this process an appropriate substrate, i.e. HHL is used; it is adapted to be hydrolysed by the ACE enzyme forming a C-terminal dipeptide (the same released by the ACE enzyme in converting the angiotensin I into angiotensin II) and hippuric acid or benzoylglycine, which is used for detection of the hydrolysis amount carried out by ACE.

In fact, hippuric acid structure is characterised by a chromophoric group (the benzoyl group), i.e. an unsaturated group having a characteristic absorption in the ultraviolet or visible region.

In detail, the hippuric acid is characterised by a radiation absorption in wavelengths between 210 nm and 260 nm and in particular it has the maximum absorption at a wavelength of 228 nm.

The evaluation process is performed by carrying out the analysis by liquid chromatography coupled to a spectrophotometric detector of three types of different samples: a sample (C) in which in addition to HHL, only the compounds having hypotensive activity 10 are present; a series of samples (X) in which, in addition to HHL, both the compounds having hypotensive activity 10 at different concentrations and ACE- at a fixed concentration are present; and a sample (0) in which the compounds having hypotensive activity 10 are absent and in addition to HHL, only the ACE enzyme is present.

In each of said tests, the samples have been submitted to a liquid-chromatography separation in which 228 nm has been used as the detection wavelength; it corresponds to the maximum absorption of the chromophoric group of hippuric acid. In detail, the area subtended to the chromatographic peak corresponding to the hippuric acid is proportional to the activity of ACE. Once the areas relating to the concentration of hippuric acid formed in the different samples have been determined, through a suitable combination of the collected results, the inhibition percentage of ACE is drawn. In particular, the formula used for determining the inhibition percentage of ACE is the following:

$$\% \text{ ACE inhibition} = [(A_0 - A_x)/(A_0-C)] * 100$$

wherein $A_0$ indicates the peak area corresponding to hippuric acid of sample 0 in which, in addition to the HHL substrate, only the ACE enzyme is present; $A_x$ indicates the peak area corresponding to the hippuric acid of samples X in which the ACE enzyme and the compounds having hypotensive activity 10 are present in a varying ratio; and C the peak area corresponding to the hippuric acid of sample C in which, in addition to HHL, only the compounds having hypotensive activity 10 at a concentration equal to the maximum concentration of samples X are present.

In particular, for each compound having hypotensive activity 10 obtained with enzymes from different species of Lupinus, a plurality of concentrations in the reaction mixture corresponding to different inhibition percentages of ACE have been evaluated.

Once the inhibition percentage of ACE has been evaluated for each concentration value of the compounds having hypotensive activity 10, these values have been graphically reproduced in a Cartesian plane in which x represents the concentration of the compounds having hypotensive activity 10 on a logarithm base and y represents corresponding inhibition percentage of ACE.

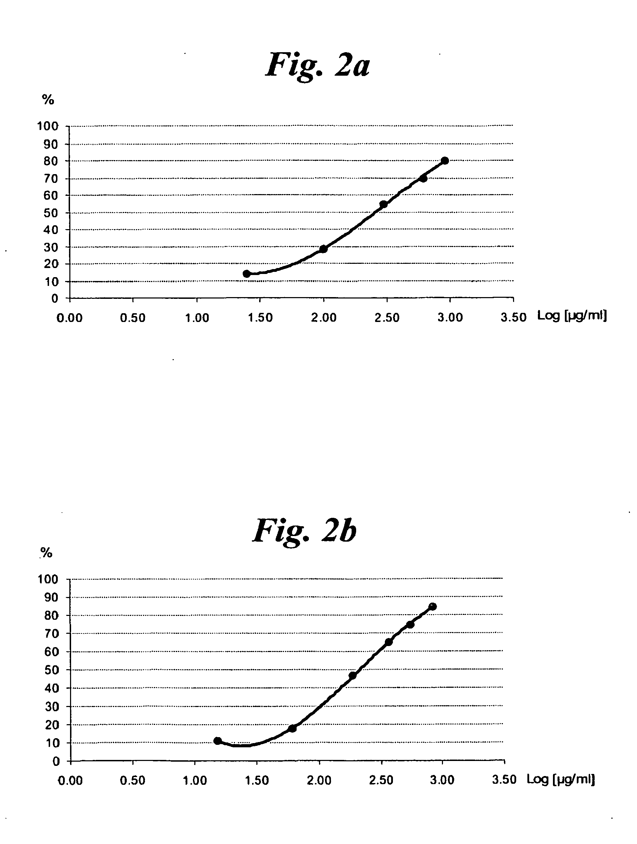

In particular, in Figs. 2a and 2b the graphs obtained using said process have been reproduced; these graphs are related to the evaluation of two compounds having hypotensive activity 10 obtained using pepsin as the enzyme 18 and *Lupinus albus* seeds and *Lupinus angustifolius* seeds respectively, as the Lupinus seeds 11.

The evaluation process is therefore completed by determining, through analysis of said graphs, which is the concentration of the compounds having hypotensive activity 10 that gives an inhibition of the ACE enzyme equal to 50%.

In particular, Table 1, reproduced below, shows the obtained maximum percentage of ACE-inhibition and the concentration of the compounds having hypotensive activity 10 required for having an inhibition of the ACE enzyme equal to 50% for some exemplary compounds 10 obtained with the production process 1.

Table 1: examples of the reached results.

| Lupinus species | Enzyme used | Maximum inhibition % | Average IC$_{50}$ [μg/mL] |
|---|---|---|---|
| Angustifolius | Corolase® PP | 65.0 | 242.2 |
| | Chymotrypsin | 79.3 | 198.6 |
| | Pepsin | 86.5 | 162.1 |
| Albus | Chymotrypsin | 73.4 | 297.2 |
| | Pepsin | 79.8 | 258.6 |

It should be pointed out that from this analysis the compounds having hypotensive activity 10 obtained through processing of *Lupinus angustifolius* seeds with chymotrypsin and pepsin appear to be particularly active; in fact, for obtaining an enzymatic inhibition of 50% (IC$_{50}$) a particularly low concentration of same is required and, more specifically, a concentration of 198.6 μg/mL and 162.1 μg/mL, respectively.

The invention proposes a new use of the compounds having hypotensive activity 10, i.e. the peptide mixture obtained from the Lupinus seeds 11 by the production process 1, for treating disorders connected with the ACE enzyme activity such as hypertension, post-infarction and chronic heart failure.

In fact, as highlighted by the aforesaid evaluation process, the compounds having hypotensive activity 10 obtained from Lupinus seeds 11 processed following process 1 enable preparation not only of foods having ACE-inhibiting activity, but also of dietary supplements.

In detail, the compounds having hypotensive activity 10 are not only used for producing food, but also for production of dietary supplements adapted to prevent and/or treat disorders connected with ACE activity.

The invention enables achievement of important advantages.

In fact, as shown by the aforesaid evaluation process and in particular in Table 1, the compounds having hypotensive activity 10 obtained by the production process 1 are particularly efficient in ACE inhibition.

These satisfactory results have been obtained by using the Lupinus seeds 11 in an advantageous and innovative manner for producing peptides having ACE-inhibitory activity.

Another advantage resulting from using of Lupinus is represented by the fact that, unlike other legumes such as soybean, peas or beans, it lacks isoflavones, molecules provided with weak estrogenic activity, whose prolonged intake over time may cause interaction with the hormonal system.

This advantage therefore enables compounds having hypotensive activity 10 which can be used for the production of foods, drinks or dietary supplements with ACE-inhibitory activity that are surely not contaminated by isoflavones.

A further advantage given by the use of Lupinus seeds 11, is that these compounds having hypotensive activity 10 are characterised by high solubility in water, stability to heat and resistance to precipitation at given pH values and in the presence of metal ions, and that therefore they are characterized by high workability and ease of use in producing foods and drinks, dietary supplements or other similar products.

Another important advantage is that the production process 11 enables to obtain an great amount of compounds having hypotensive activity 10 of high quality. This advantage is substantially due to the performing conditions of the hydrolysis step 4 and, in particular, the enzymatic-hydrolysis sub-step 4a. In fact, the above described particular selection of the parameters of the enzymatic-hydrolysis sub-steps 4a enables a high fragmentation of the proteins and a production, at the end of each individual sub-step 4a, of peptides of molecular weight lower than 10000 Da.

Therefore, at the end of the enzymatic-hydrolysis sub-step 4a a very high amount of peptides characterised by molecular weight lower than 3000 Da and provided with good ACE-inhibitory activity ($IC_{50}$ lower than 300 $\mu$g/mL) are obtained.

A further advantage is due to selection of the size of the peptides that, being smaller than 3000 Da, are easily absorbable so as to fast inhibit the ACE.

In particular, due to the low molecular weight of peptides having a very short amino acid chain, the possibility that during digestion other enzymes may further fragment the peptides causing loss of the ACE-inhibitory action can be greatly reduced.

Furthermore, a molecular weight lower than 3000 Da causes the peptides to have sufficiently reduced sizes so that they can be easily carried from the intestine to the inside of the bloodstream and therefore can quickly reach the target organ.

In fact, this molecular weight enables the peptides to use specific transport mechanisms such as paracellular and transcellular passage, admission to the enterocytes by endocytosis and crossing of the basal lateral membrane of the enterocytes through peptide carriers exclusively facilitating transport of peptides of reduced sizes into the blood.

**Claims**

1. A production process (1) for compounds having hypotensive activity, adapted to be used for preparing foods, drinks and dietary supplements; said production process (1) being adapted to obtain compounds having hypotensive activity (10) from processing/working seeds of leguminous plants and comprising an extraction step (3) in which a protein solution (13) is obtained from said seeds; and **characterised in that** said seeds of said leguminous plants are Lupinus seeds (11); and **in that** it comprises a hydrolysis step (4) in which the proteins of said protein solution (13) are fragmented thus obtaining said compounds having hypotensive activity (10) which substantially consist of peptides of a molecular weight lower than 3000 Da and having an aromatic and hydrophobic amino acid as the terminal group so as to determine an $IC_{50}$ lower than 1000 $\mu$g/mL.

2. A production process (1) as claimed in claim 1, wherein said terminal group is phenylalanine.

3. A production process (1) as claimed in claim 1, wherein said terminal group is proline.

4. A production process (1) as claimed in claim 1, wherein said terminal group is tryptophan.

5. A production process (1) as claimed in claim 1, wherein said terminal group is tyrosine.

6. A production process (1) as claimed in one or more of the preceding claims, wherein said peptides have an $IC_{50}$ lower than 300 $\mu$g/mL.

7. A production process (1) as claimed in one or more of the preceding claims, wherein said hydrolysis step (4) comprises at least one enzymatic-hydrolysis sub-step (4a) in which the proteins present in said protein solution (13) are submitted to scission by enzymatic hydrolysis and wherein from said protein solution (13) a peptide solution (17) is obtained which has a molecular weight lower than 10000 Da.

8. A production process (1) as claimed in the preceding claim, wherein in said at least one enzymatic-hydrolysis sub-step (4a) said protein solution (13) is mixed with enzymes (18) in a mixing ratio substantially included between 1/20 w/w and 1/200 w/w.

9. A production process (1) as claimed in one or more of claims 6-8, wherein in said at least one enzymatic-hydrolysis sub-step (4a) said enzymes (18) are proteolytic enzymes.

10. A production process (1) as claimed in one or more of claims 6-8, wherein in said at least one enzymatic hydrolysis sub-step (4a) said enzymes (18) are immobilised enzymes.

11. A production process (1) as claimed in one or more of the preceding claims, wherein said protein solution (13) obtained in said extraction sub-step (3) is a globular protein solution including globulins.

12. A production process (1) as claimed in one or more of the preceding claims, wherein said Lupinus seeds (11) are *Lupinus albus* seeds.

13. A production process (1) as claimed in one or more of claims 1-12, wherein said Lupinus seeds (11) are *Lupinus angustifolius* seeds.

14. A compound having hypotensive activity (10) adapted to be used for preparation of foods, drinks and dietary supplements, **characterised in that** it is produced from Lupinus seeds (11) and **in that** it comprises peptides of a molecular weight lower than 3000 Da and having an aromatic or hydrophobic amino acid as the terminal group so as to determine an $IC_{50}$ lower than 1000 $\mu$g/mL.

15. A compound having hypotensive activity (10) as claimed in the preceding claim, wherein said peptides have an $IC_{50}$ lower than 300 $\mu$g/mL.

**Patentansprüche**

1. Verfahrensprozess zur Herstellung (1) von Verbindungen mit hypotonischer Wirkung, die geeignet sind für die Zubereitung von Lebensmitteln, Getränken und Nahrungsergänzungsmitteln verwendet zu werden; wobei der genannte Verfahrensprozess (1) geeignet ist, Verbindungen mit hypotonischer Wirkung (10) aus der Verarbeitung von Samen von Hülsenfrüchtlern zu gewinnen und eine Extraktionsphase (3) umfasst, in der aus den genannten Samen eine eiweißhaltige Lösung (13) gewonnen wird und **dadurch gekennzeichnet ist, dass** es sich bei den genannten Samen der genannten Hülsenfrüchtler um Lupinensamen (11) handelt und dadurch, eine Hydrolysephase (4) zu beinhalten, in der die Proteine der genannten eiweißhaltigen Lösung (13) zerkleinert und so die genannten Verbindungen mit hypotonischer Wirkung (10) erzielt werden, die sich im Wesentlichen aus Peptiden mit einem Molekulargewicht von unter 3000 Da zusammensetzen und als Endgruppe eine aromatische und hydrophobe Aminosäure aufweisen, so dass ein $IC_{50}$ unter 1000 $\mu$g/mL erreicht wird.

2. Verfahrensprozess zur Herstellung (1) nach Anspruch 1, bei dem die genannte Endgruppe Phenylalanin ist.

3. Verfahrensprozess zur Herstellung (1) nach Anspruch 1, bei dem die genannte Endgruppe Prolin ist.

4. Verfahrensprozess zur Herstellung (1) nach Anspruch 1, bei dem die genannte Endgruppe Tryptophan ist.

5. Verfahrensprozess zur Herstellung (1) nach Anspruch 1, bei dem die genannte Endgruppe Thyrosin ist.

6. Verfahrensprozess zur Herstellung (1) nach Anspruch 1, bei dem die genannten Peptide ein $IC_{50}$ unter 300 $\mu$g/mL aufweisen.

7. Verfahrensprozess zur Herstellung (1) nach einem oder mehreren der vorangegangenen Ansprüche, bei dem die genannte Hydrolysephase (4) mindestens eine enzymatische Unterphase der Hydrolyse (4a) umfasst, bei der die in der genannten Eiweißlösung (13) enthaltenen Proteine durch enzymatische Hydrolyse aufgespalten werden und bei der aus der genannten Eiweißlösung (13) eine Peptidlösung (17) mit einem Molekulargewicht von unter 10000 Da gewonnen wird.

8. Verfahrensprozess zur Herstellung (1) nach dem vorangegangenen Anspruch, bei dem in der genannten mindestens einen enzymatischen Unterphase der Hydrolyse (4a), die genannte Eiweißlösung (13) mit Enzymen (18) in einem Mischverhältnis gemischt wird, das im Wesentlichen zwischen 1/20 p/p und 1/200 p/p liegt.

9. Verfahren zur Herstellung (1) nach einem oder mehreren der Ansprüche 6-8, bei dem in der mindestens einen enzymatischen Unterphase der Hydrolyse (4a) die genannten Enzyme (18) proteolytisch sind.

10. Verfahren zur Herstellung (1) nach einem oder mehreren der Ansprüche 6-8, bei dem es sich in der genannten mindestens einen enzymatischen Unterphase der Hydrolyse (4a) bei den genannten Enzymen (18) um immobilisierte Enzyme handelt.

11. Verfahren zur Herstellung (1) nach einem oder mehreren der vorangegangenen Ansprüche, bei dem die genannten Eiweißlösung (13), die in der genannten Extraktionsphase (3) erhalten wurde, eine globulare Eiweißlösung ist, die Globuline umfasst.

12. Verfahren zur Herstellung (1) nach einem oder mehreren der vorangegangenen Ansprüche, bei dem die genannten Lupinensamen (11) Samen von Lupinus albus sind.

13. Verfahren zur Herstellung (1) nach einem oder mehreren der Ansprüche 1-12, bei dem die genannten Lupinensamen (11) Samen von Lupinus angustifolius sind.

14. Verbindung mit hypotonischer Wirkung (10), die geeignet ist, zur Zubereitung von Lebensmitteln, Getränken und Nahrungsergänzungsmitteln verwendet zu werden und **dadurch gekennzeichnet, dass** sie aus Lupinensamen (11) hergestellt wird und dadurch, Peptide mit einem Molekulargewicht von unter 3000 Da zu enthalten und als Endgruppe eine aromatische und hydrophobische Aminosäure aufzuweisen, so dass sich ein $IC_{50}$ von unter 1000 $\mu g/mL$ ergibt.

15. Verbindung mit hypotonischer Wirkung (10) nach dem vorangegangenen Anspruch, bei dem die genannten Peptide einen $IC_{50}$ unter 300 $\mu g/mL$ aufweisen.

**Revendications**

1. Procédé de fabrication (1) de composés dotés d'une activité hypotensive aptes à être utilisés pour la préparation d'aliments, de boissons et de compléments alimentaires ; ledit procédé de fabrication (1) étant apte à obtenir des composés dotés d'une activité hypotensive (10) du traitement de graines de plantes légumineuses et comprenant une phase d'extraction (3) dans laquelle on obtient de ces graines une solution protéique (13) ; et **caractérisé en ce que** lesdites graines desdites plantes légumineuses sont des graines de Lupinus (11) ; et **en ce qu'**il comprend une phase d'hydrolyse (4) dans laquelle les protéines de ladite solution protéique (13) sont fragmentées en obtenant lesdits composés dotés d'une activité hypotensive (10) fondamentalement constitués de peptides ayant un poids moléculaire inférieur à 3 000 Da et ayant comme groupe terminal un acide aminé aromatique et hydrophobe tel à déterminer un $IC_{50}$ inférieur à 1 000 $\mu g/mL$.

2. Procédé de fabrication (1) selon la revendication 1, dans lequel ledit groupe terminal est phénylalanine.

3. Procédé de fabrication (1) selon la revendication 1, dans lequel ledit groupe terminal est proline.

4. Procédé de fabrication (1) selon la revendication 1, dans lequel ledit groupe terminal est tryptophane.

5. Procédé de fabrication (1) selon la revendication 1, dans lequel ledit groupe terminal est tyrosine.

6. Procédé de fabrication (1) selon une ou plusieurs des revendications précédentes, dans lequel lesdits peptides ont $IC_{50}$ inférieur à 300 $\mu g/mL$.

7. Procédé de fabrication (1) selon une ou plusieurs des revendications précédentes, dans lequel ladite phase d'hydrolyse (4) comprend au moins une sous-phase d'hydrolyse enzymatique (4a) dans laquelle les protéines présentes dans ladite solution protéique (13) sont scindées par hydrolyse enzymatique et dans laquelle on obtient de ladite solution protéique (13) une solution peptidique (17) avec poids moléculaire inférieur à 10 000 Da.

8. Procédé de fabrication (1) selon la revendication précédente, dans lequel, dans ladite au moins une sous-phase d'hydrolyse enzymatique (4a), ladite solution protéique (13) est mélangée à des enzymes (18) avec un rapport de mélange fondamentalement compris entre 1/20 p/p et entre 1/200 p/p.

9. Procédé de fabrication (1) selon une ou plusieurs des revendications 6-8, dans lequel, dans ladite au moins une sous-phase d'hydrolyse enzymatique (4a), ledites enzymes (18) sont protéolytiques.

10. Procédé de fabrication (1) selon une ou plusieurs des revendications 6-8, dans lequel, dans ladite au moins une sous-phase d'hydrolyse enzymatique (4a), lesdites enzymes (18) sont des enzymes immobilisées.

11. Procédé de fabrication (1) selon une ou plusieurs des revendications précédentes, dans lequel ladite solution protéique (13), obtenue dans ladite phase d'extraction (3), est une solution protéique globulaire comprenant des globulines.

12. Procédé de fabrication (1) selon une ou plusieurs des revendications précédentes, dans lequel lesdites graines de Lupinus (11) sont des graines de Lupinus albus.

13. Procédé de fabrication (1) selon une ou plusieurs des revendications 1-12, dans lequel lesdites graines de Lupinus (11) sont des graines de Lupinus angustifolius.

14. Composé doté d'activité hypotensive (10) apte à être utilisé pour la préparation d'aliments, de boissons et de compléments alimentaires **caractérisé en ce qu'**il est fabriqué à partir de graines de Lupinus (11) et **en ce qu'**il comprend des peptides avec poids moléculaire inférieur à 3 000 Da et ayant comme groupe terminal un acide aminé aromatique et hydrophobe tel à déterminer un $IC_{50}$ inférieur à 1 000 $\mu$g/mL.

15. Composé doté d'activité hypotensive (10) selon la revendication précédente, dans lequel lesdits peptides ont $IC_{50}$ inférieur à 300 $\mu$g/mL.

Fig. 1

EP 2 548 458 B1

## *Fig. 2a*

## *Fig. 2b*

**REFERENCES CITED IN THE DESCRIPTION**

**Patent documents cited in the description**

- KR 100818010 B1
- US 2008050458 A1
- JP 2009073765 B
- JP 2007217406 B
- EP 1374885 A